(19) **Europäisches Patentamt**

**European Patent Office**

**Office européen des brevets**

(11) **EP 1 229 048 A1**

(12) **EUROPEAN PATENT APPLICATION**

(43) Date of publication:
**07.08.2002 Bulletin 2002/32**

(51) Int Cl.⁷: **C07K 14/745**, A61K 38/36,
A61K 47/18, G01N 33/86

(21) Application number: **02001985.7**

(22) Date of filing: **04.02.2002**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **05.02.2001 JP 2001028828**

(71) Applicant: **INTERNATIONAL REAGENTS CORPORATION**
**Kobe-shi Hyogo 651-0083 (JP)**

(72) Inventors:
• **Kikukawa, Norihiro,**
**International Reagents Corp.**
**Kobe-shi, Hyogo 651-2241 (JP)**
• **Okuda, Masahiro, International Reagents Corp.**
**Kobe-shi, Hyogo 651-2241 (JP)**

(74) Representative:
**Forstmeyer, Dietmar, Dr. rer. nat., Dipl.-Chem. et al**
**Boeters & Bauer,**
**Bereiteranger 15**
**81541 München (DE)**

(54) **Thromboplastin reagent and method for manufacturing the same**

(57)    This invention provides a novel thromboplastin reagent having high measurement sensitivity. Thus, an effective amount of amino acid or derivative thereof having such a function that an ISI (international sensitivity index) of a composition of thromboplastin showing an ISI of more than 1.0 is made nearer 1.0 is added whereby a thromboplastin having a high measurement sensitivity is provided. To be more specific, sodium glutamate is added in an effective amount.

**Description**

**Background of the Invention**

**1. Field of the Invention**

The present invention relates to a thromboplastin reagent used for measurement of a coagulation factor where a novel reagent having high sensitivity is provided and also relates to a method for manufacturing the same.

**2. Description of the Related Art**

As a test for screening the defect of coagulation factors in the blood of patients, Quick's Prothrombin time (PT) has been used. PT is also used as a monitor for the therapy by oral anticoagulant therapy. Although measurement of the PT varies depending upon the property of the thromboplastin reagent, PT of normal blood donor is 10-14 seconds and, with regard to the PT of plasma deficient in coagulation factors, it is preferred to use a thromboplastin reagent which is prepared in such a manner that the PT can be prolonged depending upon the degree of defect of the coagulation factor.

Active thromboplastin induces the coagulation in plasma and is composed of a lipid component and a protein component. Protein, i.e. the tissue factor, is bonded to membrane and is found in many various tissues. The bond between the protein and the lipid is independent on $Ca^{2+}$ due to a hydrophobic interaction. Protein residue comprises glycoprotein having a molecular weight of 43-53 kDa. One molecule of the tissue factor is able to bond to one molecule of a coagulation factor VII or a coagulation factor VIIa. Bond of the coagulation factor VII/coagulation factor VIIa to the tissue factor is dependent on $Ca^{2+}$. A complex comprising lipid, tissue factor and coagulation factor VIIa cleaves a coagulation factor X to form a coagulation factor Xa whereby blood coagulation by the activated prothrombin is finally induced.

Initiation of coagulation in plasma after 10-14 seconds from addition of a thromboplastin reagent indicates that the coagulation system is unhurt. An increase in the coagulation time causes a certain disorder. The disorder occurs as a result of too low concentration of one or more coagulation factor(s).

Thromboplastin can be extracted from many kinds of tissues of various animal materials. Due to the limited availability, the cost thereof, etc., materials which are generally utilized are limited and thromboplastin derived from rabbit brain which is the most common material has a relatively low sensitivity as compared with thromboplastin derived typically from human tissues. Materials, extracting method and reagent composition for thromboplastin are important factors for determining the sensitivity of the reagent. Under such circumstances, in order to improve the sensitivity of thromboplastin, investigation for extraction using a nonionic detergent or the like has been attempted and reported (Japanese Patent Laid-Open No. 03/503534).

As to another method for improving the sensitivity, there has been reported a method where a small amount of protein is specifically removed. It is often that thromboplastins of various origins are different in terms of their sensitivity indicating the defect of specific coagulation factors. In some cases, that is due to the fact that a small amount of the factor to be measured is brought into the reagent.

For example, there is a report for a method of improving the sensitivity by a selective inhibition of coagulation factor VII/coagulation factor VIIa remaining in the thromboplastin reagent (Japanese Patent Laid-Open No. 10/330,400).

For the measurement of a more correct coagulation time, standardization of the PT measurement has been carried out. For such a purpose, a standard sample of a thromboplastin reagent is prepared, sensitivity of each reagent is expressed in terms of international sensitivity index (hereinafter, referred to as "ISI") on the basis of the above and that is described for each reagent.

When PT is measured using a thromboplastin reagent, ISI value becomes low when the difference between the PT of normal human plasma and the measured PT of plasma deficient in coagulation factor is big while, when the said different is small, ISI value becomes high. Accordingly, the ISI value of a thromboplastin reagent having high measurement sensitivity is low and there has been a demand for such a reagent. The ISI value of the international standard preparation of thromboplastin is 1.0.

However, as mentioned already, property of a thromboplastin reagent varies depending upon the material composition therefor. For example, the property differs when the material is derived from human being, rabbit or bovine. There are many cases where there is prepared a composition containing a thromboplastin showing an ISI value of more than 1.0.

Further, in some cases, a step of freeze-drying treatment during the manufacturing steps of a thromboplastin reagent affects the measurement of the coagulation time. Thus, when the PT is measured using the said freeze-dried thromboplastin reagent, there are some cases where it is longer than 14 seconds even when normal plasma is used and such a phenomenon results from a freeze-drying process. The use of a thromboplastin reagent showing a value of longer than 14 seconds in the case of normal plasma is not preferred in view of efficiency of the measurement and there has been also a demand for development of a manufacturing method whereby the damage by freeze-drying failure is excluded.

## Summary of the Invention

The matter to be solved by the present invention is to provide a novel thromboplastin reagent having high measurement sensitivity.

The present inventors have carried out an intensive investigation by paying their attention to the ISI value and have found that, when amino acid or amino acid derivative is added to a thromboplastin-containing composition having an ISI value of more than 1.0, there are some cases where the ISI value becomes nearer 1.0. The present invention has been achieved by addition of amino acid or amino acid derivative having such a function in an effective amount to thromboplastin. It has been further investigated for the stage when such an amino acid or amino acid derivative is to be added and, as a result, it has been found that a stable thromboplastin reagent having no reduction in the activity by freeze-drying can be provided whereupon the present invention has been achieved.

Thus, the present invention comprises the followings.

1. A method for the manufacture of a thromboplastin reagent which is characterized in that, in the manufacturing steps of thromboplastin reagent, there is included a step where an effective amount of amino acid or derivative thereof having such a function that an ISI (international sensitivity index) of a thromboplastin-containing composition showing an ISI of more than 1.0 is made nearer 1.0 is added.

2. The method according to the above 1, wherein, as the effective amount of amino acid or derivative thereof mentioned in the above 1, it is added so as to make the final concentration 0.01-20 w/v%.

3. The method according to the above 1 or 2, wherein the amino acid or derivative thereof mentioned in the above 1 is glutamic acid, sodium glutamate or glycine.

4. The method according to any of the above 1 to 3, wherein the step of addition of the amino acid or derivative thereof is after the step for the extraction of thromboplastin from the material composition and is before the freeze-drying.

5. The method according to any of the above 1 to 3, wherein the step of addition of the amino acid or derivative thereof is after the step for the extraction of thromboplastin from the material composition and for the freeze-drying.

6. A thromboplastin reagent which is manufactured by any of the methods mentioned in the above 1 to 5.

7. A thromboplastin reagent, characterized in that, there is contained an effective amount of an amino acid or derivative thereof which has a function that an ISI (international sensitivity index) of a thromboplastin-containing composition showing an ISI of more than 1.0 is made nearer 1.0.

8. The thromboplastin reagent according to the above 7, wherein, as the effective amount of amino acid or derivative thereof mentioned in the above 7, it is added so as to make the final concentration 0.01-20 w/v%.

9. The thromboplastin reagent according to the above 7 or 8, wherein the amino acid or derivative thereof mentioned in the above 7 is glutamic acid, sodium glutamate or glycine.

10. A kit for the measurement of coagulation time containing the thromboplastin reagent described in any of the above 6 to 9.

## Detailed Description of the Preferred Embodiments

INR (international normalized ratio) is used as a new way of describing a PT. The INR value is calculated by rising to ISI power of a prothrombin ratio (PR) and its normal value is 1.0. Here, a prothrombin ratio is the ratio of the PT of normal plasma to the PT of patient plasma and is expressed by PR.

The relation between INR and ISI is given by the following formula.

$$INR = PR^{ISI} = [(PT \text{ of patient plasma})/(PT \text{ of normal plasma})]^{ISI}$$

Thus, when the ISI value is high in a thromboplastin reagent, it is a reagent where the difference between the PT of normal plasma and the PT of plasma deficient in a coagulation factor is little while, when the ISI value therein is low, it is a reagent where the difference between the PT of normal plasma and the PT of plasma deficient in a coagulation factor is large. Therefore, it is believed that, when the PT changes depending upon the amount of the coagulation factor contained therein, amount of the coagulation factor can be measured with a good precision.

At present, various kinds of thromboplastin-containing compositions extracted from human placenta, rabbit brain, bovine brain, etc. are used as bulk materials for thromboplastin reagent and most of such compositions show an ISI of more than 1.0. Thus, when the ISI is made nearer 1.0, it is possible to provide a thromboplastin reagent having a suitable sensitivity. Such a way of thinking is not limited to a thromboplastin reagent derived from natural substances only but is applicable to thromboplastin reagents prepared by means of a recombinant technology as well. In the present invention, a thromboplastin-containing composition may be anything so far as it contains thromboplastin and there is no limitation for its source. For example, thromboplastin (tissue factor)-containing compositions not only derived from natural substances such as human placenta, rabbit brain and bovine brain but also prepared by means of a recombinant technology, etc. are included.

An amino acid or derivative thereof having a function of making an ISI of a thromboplastin-containing composition which has an ISI of more than 1.0 nearer 1.0 is a substance which has a function of lowering the ISI value to make nearer 1.0 when an appropriate amount is added to a thromboplastin composition and it stands for an amino acid or derivative thereof. Examples of the amino acid or derivative thereof having such a function are alanine, aminobutyric acid (hereinafter, referred to as "ABA"), glutamic acid, glutamine, sodium glutamate, glycine, methionine, proline, serine and tyrosine. Preferably, they are alanine, aminobutyric acid, sodium glutamate, glutamic acid or glycine. And a more preferred example is sodium glutamate, glutamic acid or glycine.

With regard to an effective amount of the amino acid or derivative thereof having the above-mentioned function, there is no particular limitation so far as it is an amount achieving a function whereby the ISI value is lowered to make nearer 1.0. For example, in terms of the final concentration in a thromboplastin reagent, a range of 0.01-20 w/v%, preferably 0.1-10 w/v% or, more preferably, 0.5-5 w/v% may be exemplified.

The amino acid or derivative thereof having the above-mentioned function may be added at any stage during the manufacturing steps and there is no particular- limitation therefor. It is also possible that, after being prepared as a reagent composition, the above-mentioned effective amount is added. Especially when the amino acid or derivative thereof having the above-mentioned function is added with an object of preventing the reduction of activity caused by the so-called freeze-drying, it is preferred to add prior to the freeze-drying during the manufacturing steps.

In addition to the freeze-dried product, the thromboplastin reagent of the present invention may be in a form of a liquid product or a frozen product.

**Examples**

The present invention will now be further illustrated by way of the following Examples although the present invention is not limited thereto.

Example 1.

PT values when various concentrations of various kinds of amino acid or derivatives thereof were added to thromboplastin bulk solution (derived from rabbit brain) prepared by a method described in Japanese Patent Laid-Open No. 05/60,762 were measured and then ISI value for each of the sample was determined on the basis of calibration plasma where an INR was previously regulated.

Measurement of the PT was carried out according to a known measuring method.

To be more specific, measurement was conducted by the following method. Thus, each of various amino acids or amino acid derivatives was added to a thromboplastin bulk solution so as to make its final concentration 1, 2 or 3 w/v%, then a calcium salt was added thereto to make its final concentration 10 mM and the mixture was freeze-dried to give a thromboplastin sample. Calibration plasma (0.05 ml) was pipeted and incubated at 37°C for about 1 minute. To this was added 0.1 ml of a thromboplastin reagent solution which was previously reconstituted in pure water and mixed with calcium in a concentration of 10 mM incubated at 37°C and then PT was measured using a Coagrex-700 (an automated coagulation analyzer; manufactured by Shimadzu).

An ISI value of each thromboplastin sample solution was determined from the measured PT value and the INR value of the calibration plasma and influence of each additive on ISI was tested.

The result was shown in Table 1.

As a result, with regard to a calibration plasma (AK-A), PT was shortened in all cases where a thromboplastin reagent to which amino acid or derivative thereof was added was used as compared with the case of the use of thromboplastin reagent to which no additive was added. Thus, it is likely that, when various additives are added to a thromboplastin bulk reagent, the so-called reduction in the activity by freeze-drying can be prevented whereby it is possible to provide a reagent having a high stability.

In the case of a calibration plasma (AK-D), PT was prolonged except the case where 1% alanine was added. It suggests that PT is elongated depending upon a reduction in the content of the coagulation factor contained in the calibration plasmas (AK-A~D) and accordingly that measurement with better sensitivity is possible.

The ISI value of each thromboplastin reagent was smaller in all cases than the ISI value of a thromboplastin to which no additive was added.

Table 1

| Prothrombin Time (PT) and ISI Values when Various Additives were Added (PT: seconds) | | | | | |
|---|---|---|---|---|---|
| | INR | Nothing Added | 1% Glu · Na | 2% Glu · Na | 3% Glu · Na |
| AK-A | 1.04 | 14.3 | 13.6 | 13.5 | 13.9 |
| AK-B | 1.92 | 20.4 | 20.8 | 21.7 | 23.4 |
| AK-C | 3.07 | 27.1 | 28.4 | 30.1 | 33.5 |
| AK-D | 4.37 | 33.3 | 35.8 | 37.6 | 41.5 |
| ISI = | | 1.69 | 1.48 | 1.4 | 1.3 |
| | INR | Nothing Added | 1% Ala | 2% Ala | 3% Ala |
| AK-A | 1.04 | 14.3 | 13.5 | 13.9 | 13.6 |
| AK-B | 1.92 | 20.4 | 19.9 | 20.9 | 20.6 |
| AK-C | 3.07 | 27.1 | 27.1 | 28.2 | 28.3 |
| AK-D | 4.37 | 33.3 | 33.1 | 34.8 | 35.3 |
| ISI = | | 1.69 | 1.59 | 1.56 | 1.5 |
| | INR | Nothing Added | 1% ABA | 2% ABA | 3% ABA |
| AK-A | 1.04 | 14.3 | 13.5 | 13.6 | 13.5 |
| AK-B | 1.92 | 20.4 | 20.3 | 20.9 | 21.0 |
| AK-C | 3.07 | 27.1 | 27.4 | 27.8 | 28.4 |
| AK-D | 4.37 | 33.3 | 34.2 | 34.6 | 35.3 |
| ISI = | | 1.69 | 1.54 | 1.54 | 1.49 |

Each of AK-A, B, C and D is calibration plasma and has an intrinsic INR value.
ISI value for each sample was calculated from the measured PT value and the calibration plasma INR value for each sample.

Example 2.

PT was measured for the case where sodium glutamate was added as an additive to a thromboplastin bulk solution to such an extent that its final concentration was 1, 2, 3, 4 or 5 w/v% and then an ISI value was determined.
Measurement of PT and calculation of ISI value were carried out in the same manner as in Example 1.
The result was shown in Table 2.

Table 2

| Prothrombin Time (PT) and ISI Values when Various Concentrations of Sodium Glutamate were Added (PT: seconds) | | | | | | | |
|---|---|---|---|---|---|---|---|
| | INR | Nothing Added | 1% Glu · Na | 2% Glu · Na | 3% Glu · Na | 4% Glu · Na | 5% Glu · Na |
| AK-A | 1.04 | 14.7 | 14.3 | 14.0 | 14.0 | 14.8 | 15.5 |
| AK-B | 1.92 | 20.5 | 21.6 | 22.4 | 24.7 | 25.9 | 28.7 |
| AK-C | 3.07 | 28.0 | 30.2 | 31.5 | 35.5 | 38.8 | 41.8 |
| AK-D | 4.37 | 35.2 | 38.6 | 41.2 | 46.4 | 50.4 | 57.5 |

Table 2 (continued)

| | | | | | | | |
|---|---|---|---|---|---|---|---|
| Prothrombin Time (PT) and ISI Values when Various Concentrations of Sodium Glutamate were Added (PT: seconds) | | | | | | | |
| | INR | Nothing Added | 1% Glu · Na | 2% Glu · Na | 3% Glu · Na | 4% Glu · Na | 5% Glu · Na |
| ISI = | | 1.64 | 1.44 | 1.33 | 1.2 | 1.16 | 1.1 |

Each of AK-A, B, C and D is calibration plasma and has an intrinsic INR value.

ISI value for each sample was calculated from the measured PT value and the calibration plasma INR value for each sample.

The ISI value for each thromboplastin sample became small depending upon the concentration of sodium glutamate and an improvement in the sensitivity of each thromboplastin sample was noted.

On the other hand, the PT for the amended plasma (AK-A) was shortest when 2 w/v% or 3 w/v% of sodium glutamate was added to give a thromboplastin reagent having a high stability.

As fully illustrated hereinabove, it is now possible to provide a thromboplastin reagent having a high measurement sensitivity and a high stability when, in the manufacturing steps of thromboplastin reagent, there is included a step where an effective amount of amino acid or derivative thereof having such a function that an ISI (international sensitivity index) of a composition of thromboplastin showing an ISI of more than 1.0 is made nearer 1.0 is added.

**Claims**

1. A method for the manufacture of a thromboplastin reagent which is **characterized in that**, in the manufacturing steps of thromboplastin reagent, there is included a step where an effective amount of amino acid or derivative thereof having such a function that an ISI (international sensitivity index) of a thromboplastin-containing composition showing an ISI of more than 1.0 is made nearer 1.0 is added.

2. The method according to claim 1, wherein, as the effective amount of amino acid or derivative thereof mentioned in the above 1, it is added so as to make the final concentration 0.01-20 w/v%.

3. The method according to claim 1 or 2, wherein the amino acid or derivative thereof mentioned in claim 1 is glutamic acid, sodium glutamate or glycine.

4. The method according to any of claims 1 to 3, wherein the step of addition of the amino acid or derivative thereof is after the step for the extraction of thromboplastin from the material composition and is before the freeze-drying.

5. The method according to any of claims 1 to 3, wherein the step of addition of the amino acid or derivative thereof is after the step for the extraction of thromboplastin from the material composition and for the freeze-drying.

6. A thromboplastin reagent which is manufactured by any of the methods mentioned in any of claims 1 to 5.

7. A thromboplastin reagent, **characterized in that**, there is contained an effective amount of an amino acid or derivative thereof which has a function that an ISI (international sensitivity index) of a thromboplastin-containing composition showing an ISI of more than 1.0 is made nearer 1.0.

8. The thromboplastin reagent according to claim 7, wherein, as the effective amount of amino acid or derivative thereof mentioned in claim 7, it is added so as to make the final concentration 0.01-20 w/v%.

9. The thromboplastin reagent according to claim 7 or 8, wherein the amino acid or derivative thereof mentioned in claim 7 is glutamic acid, sodium glutamate or glycine.

10. A kit for the measurement of coagulation time containing the thromboplastin reagent described in any of claims 6 to 9.

**European Patent Office**

## PARTIAL EUROPEAN SEARCH REPORT

**Application Number**

which under Rule 45 of the European Patent Convention
shall be considered, for the purposes of subsequent
proceedings, as the European search report

EP 02 00 1985

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 98 48283 A (INSTRUMENTATION LAB SPA) 29 October 1998 (1998-10-29) * paragraph joining pages 5 and 6; example 1; claim 15 * | 1-10 | C07K14/745 A61K38/36 A61K47/18 G01N33/86 |
| X | EP 0 241 613 A (BIO DATA CORP) 21 October 1987 (1987-10-21) * page 3, lines 33-35; page 4, line 25 – page 6, line 5 (in particular, page 5, line 6) * | 1-10 | |
| X | DE 23 16 430 A (BEHRINGWERKE AG) 10 October 1974 (1974-10-10) * page 4, lines 21-30; example 1 * | 1-10 | |
| X | WO 00 70084 A (LEE TED C K ;INTERNAT TECHNIDYNE CORP (US)) 23 November 2000 (2000-11-23) * page 4, last paragraph; tables A and B; paragraph joining pages 19 and 20; examples 1-11 * | 1-3,6-10 | |
| | -/-- | | **TECHNICAL FIELDS SEARCHED (Int.Cl.7)** C07K A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 22 March 2002 | Fausti, S |

EPO FORM 1503 03.82 (P04C07)

**European Patent Office** | **INCOMPLETE SEARCH SHEET C** | **Application Number** EP 02 00 1985

Claim(s) searched completely:
      3,9

Claim(s) searched incompletely:
      1,2,4-8,10

Reason for the limitation of the search:

Present claims 1 and 7 relate respectively to a method and a product defined (inter alia) by reference to the following parameter:
ISI (international sensitivity  index)
The use of this parameter in the present context is considered to lead to a lack of clarity within the meaning of Article 84 EPC. It is impossible to compare the parameters the applicant has chosen to employ with what is set out in the prior art.
Independent of the above reasoning, the claims also lack clarity (Article 84 EPC). An attempt is made to define the product/method by reference to a result to be achieved, namely the desired reduction of the ISI index.

The lack of clarity (Art. 84 EPC) is such as to render a meaningful complete search impossible. Consequently, the search has been restricted to methods and products wherein the "effective amount of amino acid or derivative thereof" is defined as any amount of the amino acids or derivatives mentioned in the description on page 9 (lines 1-3), i.e.:
Ala, aminobutyric acid, Glu, Gln, sodium glutamate, Gly, Met, Pro, Ser, and Tyr.

**European Patent Office**

## PARTIAL EUROPEAN SEARCH REPORT

Application Number

EP 02 00 1985

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | EP 0 107 383 A (ORTHO DIAGNOSTIC SYSTEMS INC) 2 May 1984 (1984-05-02) * page 11, lines 29 - page 12, line 16; claims 1,3-8 * | 1-3,6-10 | |
| X | PATENT ABSTRACTS OF JAPAN vol. 1995, no. 03, 28 April 1995 (1995-04-28) & JP 06 337267 A (TOKUYAMA SODA CO LTD), 6 December 1994 (1994-12-06) * abstract * | 1,3,6,7,9 | |

**TECHNICAL FIELDS SEARCHED** (Int.Cl.7)

EPO FORM 1503 03.82 (P04C10)

**EP 1 229 048 A1**

**ANNEX TO THE EUROPEAN SEARCH REPORT
ON EUROPEAN PATENT APPLICATION NO.**  EP 02 00 1985

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2002

| Patent document cited in search report | | Publication date | Patent family member(s) | | Publication date |
|---|---|---|---|---|---|
| WO 9848283 | A | 29-10-1998 | AU | 7129598 A | 13-11-1998 |
| | | | EP | 1015890 A1 | 05-07-2000 |
| | | | JP | 3246749 B2 | 15-01-2002 |
| | | | JP | 11514101 T | 30-11-1999 |
| | | | US | 6100072 A | 08-08-2000 |
| | | | WO | 9848283 A1 | 29-10-1998 |
| EP 0241613 | A | 21-10-1987 | US | 4755461 A | 05-07-1988 |
| | | | EP | 0241613 A1 | 21-10-1987 |
| | | | JP | 62250364 A | 31-10-1987 |
| | | | NO | 861882 A | 19-10-1987 |
| DE 2316430 | A | 10-10-1974 | DE | 2316430 A1 | 10-10-1974 |
| | | | FR | 2223383 A1 | 25-10-1974 |
| | | | IT | 1043913 B | 29-02-1980 |
| | | | US | 3980432 A | 14-09-1976 |
| WO 0070084 | A | 23-11-2000 | AU | 5141300 A | 05-12-2000 |
| | | | EP | 1185690 A1 | 13-03-2002 |
| | | | WO | 0070084 A1 | 23-11-2000 |
| EP 0107383 | A | 02-05-1984 | AT | 21264 T | 15-08-1986 |
| | | | AU | 564622 B2 | 20-08-1987 |
| | | | AU | 1970383 A | 05-04-1984 |
| | | | CA | 1213198 A1 | 28-10-1986 |
| | | | DE | 3365155 D1 | 11-09-1986 |
| | | | EP | 0107383 A1 | 02-05-1984 |
| | | | JP | 1965195 C | 25-08-1995 |
| | | | JP | 6098038 B | 07-12-1994 |
| | | | JP | 59091899 A | 26-05-1984 |
| JP 06337267 | A | 06-12-1994 | JP | 3095608 B2 | 10-10-2000 |

EPO FORM P0459

For more details about this annex : see Official Journal of the European Patent Office, No. 12/82

10